# EUROPEAN PATENT APPLICATION

(11) **EP 1 081 216 A1**
(43) Date of publication of application: **07.03.2001**
(21) Application number: 00117833.4
(22) Date of filing: 18.08.2000
(51) Int. Cl.: C11D 9/00, C11D 9/18, C11D 9/22, A61K 7/50, A61K 7/06

(54) **Soap**

(30) Priority: 20.08.1999 JP 27291299
(71) Applicant: Kisho Co., Ltd., Tokyo, 104-0033 (JP); Expand-Fit Inc., Tokyo, 177-0054 (JP)
(72) Inventor: Satoh, Yoshiharu, c/o Kisho Co., Ltd., Tokyo, 104-0033 (JP)
(74) Representative: VOSSIUS & PARTNER

(57) **Abstract**

According to the invention, there is provided a soap which contains charcoal and tea but contains preferably no artificial perfumery component.

## Description

The present invention relates to soaps including cosmetic solid soap, liquid body soap, face-washing foam, face-washing powder, face-cleaning pack, hair- washing shampoo, hair-washing powder and the like.

Soap has been mainly used to keep personal cleanliness. One of important purposes of the soap lies in removing unpleasant body odor. Therefore, the soaps contain a perfumery in addition to a washing or cleaning component(s).

However, there are many perfumeries to develop various fragrances, liking of which is different by each person. Some of perfumeries may cause dermatitis due to an allergy. Furthermore, stronger washing components may stimulate the skin and thus cause dermatitis.

Therefore, an object of the invention is to provide a soap containing no artificial perfumery which soap develops relatively high cleaning power. Another object of the invention is to provide a soap which is suitable for increasing the growth of hair and provides more volume to the hair.
According to the invention, the objects can be attained by a soap which comprises powdered charcoal and at least a kind of tea selected from the group consisting of powdered tea and tea extract, in addition to conventional components.

As the charcoal, wood charcoal, coal, carbonized sawdust, carbonized coconut husk, animal charcoal and the like can be listed. The charcoal may be activated. It is most preferable to use a carbomedicinalis listed in the Japanese pharmacopoeia from a sanitary view point. An amount of the charcoal occupying in the soap varies depending on specific kind or form of the soaps, but 0.01 - 5% by weight and more preferably 0.3 - 3% by weight for the solid soap, 0.01 - 5% by weight and more preferably 0.3 - 3% by weight for the hair-washing shampoo, and 0.01 - 10% by weight and more preferably 0.5 - 5% by weight for the face-cleaning pack.

As the tea, green tea, oolong tea, black tea, *houttuynia cordata* tea, adley tea, mixture of coarse green tea and roasted whole-rice, puer tea and the like can be listed. It is preferable to use the tea in the form of powder, solution extracted by hot water, ethanol, 1,3-butylene glycol, propylene glycol, glycerine, or a mixture thereof, another solution obtained by dry distillation of the raw tea in *vacuo*, or lyophilized powder thereof or the extract. An amount of the tea occupying in the soap also varies depending on kind or form of the soaps, but 0.001 - 50% by weight and more preferably 0.1 - 5% by weight.

Each of the soaps according to the invention can be prepared by using conventional raw materials and processes except that the charcoal and tea will be added during a process according to the invention. The invention will be explained in more detail with reference to Manufacturing Examples.

### Example 1 (Liquid body soap)

A liquid body soap was prepared by a conventional process and by using following raw materials.

| Component | Amount (% by weight) |
|---|---|
| Lauric acid | 10 |
| Myristic acid | 10 |
| Compound of Coconut oil fatty acids | |
| and sodium Methyltaurine | 10 |
| Potassium hydroxide | 4 |
| Glycerine | 5 |
| Caster oil hardened by polyoxyethylene (60) | 1 |
| Carbomedicinalis | 0.8 |
| Green tea extract | 0.5 |
| Methylparaben | 0.2 |
| Hydroxyethylcellulose | 0.1 |
| Purified water | remainder |
| Total | 100.0 |

The resulting soap showed abilities of cleaning body skin and removing body odor.

### Example 2 (Liquid body soap)

A liquid body soap was prepared by a conventional process and by using following raw materials.

| Components | Amount (% by weight) |
|---|---|
| L-Glutamic acid monotoriethanolamine solution of N-acylated coconut oil fatty acids | 40 |
| Sodum N-lauroyl-N-methyl-β-alanine | 20 |
| Potassium myristate | 3 |
| Diethanol amide of coconut oil fatty acids | 3 |
| 1,3-Butylene glycol | 3 |
| Ethylene glycol distearate | 1.8 |
| Carbomedicinalis | 1 |
| Black tea extract | 0.7 |
| Methylparaben | 0.2 |
| Sodium benzoate | 0.2 |
| Purified water | remainder |
| Total | 100 |

The resulting soap also showed abilities of cleaning body skin and removing body odor.

### Example 3 (Solid cosmetic soap)

A solid cosmetic soap was prepared by a conventional process and by using following raw materials.

| Component | Amount (% by weight) |
|---|---|
| Beef tallow | 20 |
| Coconut oil | 20 |
| Ethanol | 20 |
| Sugar | 9 |
| Sodium hydroxide | 6 |
| Glycerine | 4 |
| Carbomedicinalis | 1 |
| Oolong tea extract | 1 |
| Trisodium editate | 0.1 |
| Purified water | remainder |
| Total | 100 |

The resulting soap showed abilities of cleaning body skin and removing body odor.

### Example 4 (Face-washing foam)

A face-washing foam was prepared by a conventional process and by using following raw materials.

| Component | Amount (% by weight) |
|---|---|
| Potassium N-lauroyl-L-glutamate | 25 |
| Polyethylene glycol | 20 |
| 1,3-Butylene glycol | 10 |
| Stearic acid | 10 |
| Myristic acid | 10 |
| Potassium hydroxide | 6.2 |
| Phenoxyethanol | 0.5 |
| Green tea extract | 0.5 |
| Carbomedicinalis | 0.1 |
| Purified water | remainder |
| Total | 100 |

The resulting soap showed abilities of cleaning body skin and removing body odor.

### Example 5 (Face-washing powder)

A face-washing powder was prepared by a conventional process and by using following raw materials.

| Component | Amount (% by weight) |
|---|---|
| Compound of N-acylated coconut oil fatty acids and potassium L-glutaminate | 25 |
| Talc | 20 |
| Sodium N-lauroyl-L-glutaminate | 15 |
| Carbomedicinalis | 1 |
| 1,3-Butylene glycol | 1 |
| Oolong tea extract | 0.8 |
| Corn starch | remainder |
| Total | 100 |

The resulting soup showed ability of cleaning the face.

### Example 6 (Hair shampoo)

A hair shampoo was prepared by a conventional process and by using following raw materials.

| Component | Amount (% by weight) |
|---|---|
| L-Grutamic acid monotriethanolamine solution of N-acylated coconut oil fattyacids | 40 |
| Lauryldimethylamineoxide | 10 |
| Ethanolamide laurynate | 5 |
| 1,3-Butylene glycol | 3 |
| Black tea extract | 0.5 |
| Carbomedicinalis | 0.2 |
| Cationated cellulose | 0.2 |
| Methylparaben | 0.2 |
| Purified water | remainder |
| Total | 100 |

The resulting shampoo showed abilities of removing hair odor. Also, the color tone and diameter of the hair was intensified and increased after usage. Furthermore, the shampoo has an supporting effect on hair growth and leads to an increased hair volume.

### Example 7 (Hair shampoo)

A hair shampoo was prepared by a conventional process and by using following raw materials.

| Component | Amount (% by weight) |
|---|---|
| Sodium sulphate of polyoxyethylene (3) Lauryl ether | 35 |
| Alkylated glycoside | 5 |
| Diethanolamide of coconut oil fatty acids | 4 |
| Polyethylene glycol monostearate | 3 |
| Carbomedicinalis | 0.2 |
| Methylparaben | 0.2 |
| Green tea extract | 0.3 |
| Hydroxyethylcellulose | 0.1 |
| Purified water | remainder |
| Total | 100 |

The resulting shampoo showed abilities of removing hair odor. Also, the color tone and diameter of the hair was intensified and increased after usage. Furthermore, the shampoo has an supporting effect on hair growth and leads to an increased hair volume.

### Example 8 (Face-cleaning pack)

A face-cleaning pack was prepared by a conventional process and by using following raw materials.

| Component | Amount (% by weight) |
|---|---|
| Kaoline | 15 |
| Glycerine | 15 |
| 1,3-Butylene glycol | 10 |
| Dipropylene glycol | 5 |
| Talc | 5 |
| Bentonite | 2 |
| Green tea extract | 0.5 |
| Methylparaben | 0.1 |
| Purified water | remainder |
| Total | 100 |

The resulting pack showed ability of removing dirt from the pores of the skin and making the skin smooth.

The invention will now be further explained with reference to Test Examples comparing difference in effects of the soups according to the invention, each of which contains both components of the charcoal and tea extract and soaps, each of which contains neither charcoal nor tea extract or contains either one of the charcoal and tea extract.

In Tables of the following Comparative Test Examples, unit of amount on each component is % by weight and each amount of purified water and corn starch is that for making total amount of the component to 100% by weight. Each evaluation on lathering, dirt removing effect, deodorizing effect and stimulation of the soups was given as
- Good:: 4 points,
- Normal:: 3 points,
- Somewhat bad:: 2 points, and
- Bad:: 1 point,
and judged and given in the Tables as
- ⓞ :: 4.5 points or more, as mean value,
- ○ :: more than 3.5 points and less than 4.5 points, as mean value,
- △ :: more than 2.5 points and less than 3.5 points, as mean value, and
- X :: less than 2.5 points.

### Comparative Test Example 1 (Reference Examples 1-4 and Examples 9 and 10)

Body soaps (6 kinds) were prepared by a conventional process and by using raw materials listed in following Table 1, and then each of the soaps was tested to evaluate abilities or functions thereof. Results are shown in the Table.

**Table 1**

| | Reference Example | | | | Example | |
|---|---|---|---|---|---|---|
| | 1 | 2 | 3 | 4 | 9 | 10 |
| Lauric acid | 10 | 10 | 10 | 10 | 10 | 10 |
| Myristic acid | 10 | 10 | 10 | 10 | 10 | 10 |
| Compound of coconut oil fattey acids and methyl taurine | 10 | | | 10 | 10 | |
| Propylbetaine solut. of coconut oil fatty acid amide | | 10 | 10 | | | 10 |
| Glycerine | 5 | 5 | 5 | 5 | 5 | 5 |
| Potassium hydroxide | 4 | 4 | 4 | 4 | 4 | 4 |
| Caster oil hardened by polyoxyethylene (60) | 1 | | | 1 | 1 | |
| Carbomedicinalis | | 0.1 | | 10 | 0.1 | 2 |
| Green tea extract | | | 0.5 | 0.1 | 5 | 0.5 |
| Methylparaben | 0.2 | 0.2 | 0.2 | | 0.2 | |
| Phenoxyethanol | 0.3 | | 0.3 | 0.8 | 0.3 | 0.8 |
| Hydroxyethylcellulose | 0.1 | 0.1 | 0.1 | 0.1 | 0.1 | 0.1 |
| Perfumery | | Suitable amount | | | | |
| Purified water | Remainder | | | | remainder | |

| (Evaluation item) | | | | | | |
|---|---|---|---|---|---|---|
| Lathering | ⓞ | ⓞ | ⓞ | X | ⓞ | ⓞ |
| Dirt removing effect | X | △ | X | ⓞ | ○ | ⓞ |
| Deodorizing effect | X | X | △ | ⓞ | ⓞ | ⓞ |
| Stimulation | ⓞ | ○ | ○ | X | ⓞ | ⓞ |

### Comparative Test Example 2 (Reference Examples 5-8 as well as examples 11 and 12)

Liquid body soaps (6 kinds) were prepared by a conventional process and by using raw materials listed in following Table 2 and resulting soaps were tested as in Comparative Test Example 1. Results are shown in the Table.

**Table 2**

| | Reference Example | | | | Example | |
|---|---|---|---|---|---|---|
| | 5 | 6 | 7 | 8 | 11 | 12 |
| L-Glutamic acid monotriethanol amine solution of N-acylated coconut oil fatty acids | 40 | 40 | 40 | 40 | 40 | 40 |
| Sodium N-lauroyl-N-methyl-β-alanine | 10 | 10 | 10 | 10 | 10 | 10 |
| Potassium myristinate | 3 | | 3 | | 3 | |
| Coconut oil fatty acid diethanolamide | 4 | 4 | 4 | 4 | 4 | 4 |
| 1,3-Butylene glycol | 5 | 5 | 5 | 5 | 5 | 5 |
| Ethylene glycol distearate | 1.5 | 1.5 | | | 1.5 | |
| Carbomedicinalis | | 0.05 | | 10 | 0.05 | 2 |
| Green tea extract | | | 0.5 | 0.1 | 5 | 0.5 |
| Sodium benzoate | 0.2 | 0.2 | 0.2 | | 0.2 | |
| Phenoxyethanol | 0.3 | | 0.3 | 0.8 | 0.3 | 0.8 |
| Hydroxypropylcellulose | 0.1 | 0.1 | 0.1 | 0.1 | 0.1 | 0.1 |
| Perfumery | suitable amount | | | | | |
| Purified water | remainder | | | | remainder | |

| (Evaluation item) | | | | | | |
|---|---|---|---|---|---|---|
| Lathering | ○ | ○ | ○ | X | ○ | ○ |
| Dirt removing effect | X | △ | X | ⓞ | ⓞ | ⓞ |
| Deodorizing effect | X | X | △ | ⓞ | ⓞ | ⓞ |
| Stimulation | ⓞ | ○ | ○ | X | ⓞ | ⓞ |

### Comparative Test Example 3 (Reference Examples 9-12 as well as Examples 13 and 14)

Hair-washing shampoos (6 kinds) were prepared by a conventional process and by using raw materials listed in following Table 3 and resulting shampoos were tested as in Comparative Test Example 1. Results are shown in the Table.

**Table 3**

| | Reference | | Example | | Example | |
|---|---|---|---|---|---|---|
| | 9 | 10 | 11 | 12 | 13 | 14 |
| L-Glutamic acid monotriethanol amine solution of N-acylated coconut oil fatty acids | 25 | 25 | 25 | 25 | 25 | 25 |
| Lauryldimethylamineoxide | 20 | 20 | 20 | 20 | 20 | 20 |
| Lauric acid ethanol amide | 10 | 10 | 10 | 10 | 10 | 10 |
| Cationated cellulose | 0.3 | 0.8 | 0.3 | 0.3 | 0.3 | 0.3 |
| 1,3-Butylene glycol | 1 | 1 | 1 | 1 | 1 | 1 |
| Methylparaben | 0.2 | 0.2 | 0.8 | 0.2 | 0.2 | 0.2 |
| Carbomedicinalis | | 0.2 | | 10 | 0.3 | 2 |
| Black tea extract | | | 0.5 | 0.1 | 2 | 0.05 |
| Perfumery | | 0.25 | 0.25 | | | |
| Purified water | remainder | | | | remainder | |

| (Evaluation item) | | | | | | |
|---|---|---|---|---|---|---|
| Lathering | ⓞ | ⓞ | ⓞ | X | ⓞ | ⓞ |
| Dirt removing effect | X | △ | X | ⓞ | ○ | ⓞ |
| Deodorizing effect | X | △ | △ | ⓞ | ⓞ | ⓞ |
| Stimulation | ⓞ | ○ | ○ | X | ⓞ | ⓞ |

### Comparative Test Example 4 (Reference Examples 13- 16 as well as Examples 15 and 16)

Hair-washing shampoos (6 kinds) were prepared by a conventional process and by using raw materials listed in following Table 4 and resulting shampoos were tested as in Comparative Test Example 1. Results are shown in the Table.

**Table 4**

| | Reference Example | | | | Example | |
|---|---|---|---|---|---|---|
| | 13 | 14 | 15 | 16 | 15 | 16 |
| Sodium sulphate of polyoxyethylene(3) lauryl ether | 35 | 35 | 35 | 35 | 35 | 35 |
| Alkyl glicoside | 5 | 5 | 5 | 5 | 5 | 5 |
| Coconut oil fatty acid diethanlamide | 4 | 4 | 4 | 4 | 4 | 4 |
| Hydroxyethylcellulose | 0.1 | 0.1 | 0.1 | 0.1 | 0.1 | 0.1 |
| Cationated cellulose | 0.3 | 0.3 | 0.3 | 0.3 | 0.3 | 0.8 |
| Glycerine | 3 | 3 | 3 | 3 | 3 | 3 |
| Methylparaben | 0.2 | 0.2 | 0.2 | 0.2 | 0.2 | 0.2 |
| Carbomedicinalis | | 0.2 | | 10 | 0.3 | 2 |
| Black tea extract | | | 0.5 | 0.1 | 2 | 0.05 |
| Perfumery | | 0.2 | 0.2 | | | |
| Purified water | remainder | | | | remainder | |

| (Evaluation item) | | | | | | |
|---|---|---|---|---|---|---|
| Lathering | ⓞ | ⓞ | ⓞ | X | ⓞ | ⓞ |
| Dirt removing effect | X | △ | X | ⓞ | ⓞ | ⓞ |
| Deodorizing effect | X | X | △ | ⓞ | ⓞ | ⓞ |
| Stimulation | ⓞ | ⓞ | ⓞ | X | ⓞ | ⓞ |

### Comparative Test Example 5 (Reference Examples 17-20 as well as Examples 17 and 18)

Face-cleaning packs (6 kinds) were prepared by a conventional process and by using raw materials listed in following Table 5 and resulting packs were tested as in Comparative Test Example 1. Results are shown in the Table.

**Table 5**

| | Reference Example | | | | Example | |
|---|---|---|---|---|---|---|
| | 17 | 18 | 19 | 20 | 17 | 18 |
| Kaolin | 15 | 15 | 15 | 15 | 15 | 15 |
| Talc | 5 | 5 | 5 | 5 | 5 | 5 |
| Dipropylene glycol | 5 | 5 | 5 | 5 | 5 | 5 |
| 1,3-Butylene glycol | 5 | 5 | 5 | 5 | 5 | 5 |
| Bentonite | 4 | 4 | 4 | 4 | 4 | 4 |
| Glycerine | 15 | 15 | 15 | 15 | 15 | 15 |
| Methylparaben | 0.2 | 0.2 | 0.2 | 0.2 | 0.2 | 0.2 |
| Carbomedicinalis | | 0.2 | | 10 | 0.3 | 2 |
| Adley tea extract | | | 0.5 | 0.1 | 2 | 0.05 |
| Perfumery | | 0.2 | 0.2 | | | |
| Purified water | remainder | | | | remainder | |

| (Evaluation item) | | | | | | |
|---|---|---|---|---|---|---|
| Lathering | △ | ○ | ○ | ⓞ | ○ | ○ |
| Dirt removing effect | X | △ | △ | ⓞ | ⓞ | ⓞ |
| Deodorizing effect | △ | △ | ⓞ | X | ⓞ | ⓞ |
| Stimulation | ⓞ | ○ | ⓞ | X | ⓞ | ⓞ |

### Comparative Test Example 6 (Reference Examples 21-24 as well as Examples 19 and 20)

Cosmetic solid soaps (6 kinds) were prepared by a conventional process and by using raw materials listed in following Table 6 and resulting soaps were tested as in Comparative Test Example 1. Results are shown in the Table.

**Table 6**

| | Reference Example | | | | Example | |
|---|---|---|---|---|---|---|
| | 21 | 22 | 23 | 24 | 19 | 20 |
| Beef tallow | 20 | 20 | 20 | 20 | 20 | 20 |
| Coconut oil | 20 | 20 | 20 | 20 | 20 | 20 |
| Ethanol | 20 | 20 | 20 | 20 | 20 | 20 |
| Sugar | 10 | 10 | 10 | 10 | 10 | 10 |
| Sodium hydroxide | 6 | 6 | 6 | 6 | 6 | 6 |
| Glycerine | 4 | 4 | 4 | 4 | 2 | 4 |
| Trisodium edetate | 0.1 | 0.1 | 0.1 | 0.1 | 0.1 | 0.1 |
| Carbomedicinalis | | 0.2 | | 10 | 0.3 | 2 |
| Oolong tea extract | | | 0.5 | | 2 | 0.05 |
| Perfumery | | 0.2 | 0.2 | | | |
| Purified water | remainder | | | | remainder | |

| (Evaluation item) | | | | | | |
|---|---|---|---|---|---|---|
| Lathering | ⓞ | ⓞ | ⓞ | X | ⓞ | ⓞ |
| Dirt removing effect | △ | X | △ | ⓞ | ⓞ | ⓞ |
| Deodorizing effect | X | △ | △ | ⓞ | ⓞ | ⓞ |
| Stimulation | ○ | ○ | ○ | X | ○ | ○ |

### Comparative Test Example 7 (Reference Examples 25-28 as well as Examples 21 and 22)

Face-washing powders (6 kinds) were prepared by a conventional process and by using raw materials listed in following Table 7 and resulting powders were tested as in Comparative Test Example 1. Results are shown in the Table.

**Table 7**

| | Reference Example | | | | Example | |
|---|---|---|---|---|---|---|
| | 25 | 26 | 27 | 28 | 21 | 22 |
| N-acylated coconut oil fatty acids-sodium L-glutamate | 25 | 25 | 25 | 25 | 25 | 25 |
| Talc | 20 | 20 | 20 | 20 | 20 | 20 |
| Sodium N-lauroyl-L-glutamate | 15 | 10 | 10 | 10 | 10 | 10 |
| 1,3-Butylene glycol | 1 | 1 | 1 | 1 | 1 | 1 |
| Phenoxyethanol | 0.8 | 0.3 | 0.3 | 0.8 | 0.3 | 0.3 |
| Carbomedicinalis | | 0.2 | | 10 | 0.3 | 2 |
| Black tea extract | | | 0.5 | 0.1 | 2 | 0.05 |
| Perfumery | | 0.2 | 0.2 | | | |
| Corn starch | remainder | | | | remainder | |

| (Evaluation item) | | | | | | |
|---|---|---|---|---|---|---|
| Lathering | ○ | ○ | ○ | X | ○ | ○ |
| Dirt removing effect | X | △ | △ | ○ | ⓞ | ⓞ |
| Deodorizing effect | X | △ | △ | ○ | ⓞ | ⓞ |
| Stimulation | ⓞ | ⓞ | ⓞ | X | ⓞ | ⓞ |

### Comparative Test Example 8 (Reference Examples 29-32 as well as Examples 23 and 24)

Face-washing foams (6 kinds) were prepared by a conventional process and by using raw materials listed in following Table 8 and resulting foams were tested as in Comparative Test Example 1. Results are shown in the Table.

**Table 8**

| | Reference Example | | | | Example | |
|---|---|---|---|---|---|---|
| | 29 | 30 | 31 | 32 | 23 | 24 |
| Potassium N-laulyl-L-glutamate | 25 | 25 | 25 | 25 | 25 | 25 |
| Polyethylene glycol | 20 | 20 | 20 | 20 | 20 | 20 |
| 1,3-Butylene glycol | 10 | 10 | 10 | 10 | 10 | 10 |
| Stearic acid | 10 | 10 | 10 | 10 | 10 | 10 |
| Myristic acid | 10 | 10 | 10 | 10 | 10 | 10 |
| Sodium hydroxide | 6.2 | 6.2 | 6.2 | 6.2 | 6.2 | 6.2 |
| Phenoxyethanol | 0.8 | 0.8 | 0.8 | 0.8 | 0.8 | 0.8 |
| Cathomedicinalis | | 0.2 | | 8 | 0.2 | 2 |
| Black tea extract | | | 0.8 | 0.1 | 2 | 0.05 |
| Perfumery | | 0.2 | 0.2 | | | |
| Purified water | remainder | | | | remainder | |

| (Evaluation item) | | | | | | |
|---|---|---|---|---|---|---|
| Lathering | ⓞ | ○ | ○ | X | ⓞ | ○ |
| Dirt removing effect | X | △ | X | ⓞ | ⓞ | ⓞ |
| Deodorizing effect | X | X | △ | ⓞ | ⓞ | ⓞ |
| Stimulation | ⓞ | ○ | ○ | X | ⓞ | ⓞ |

## Claims

1. Soap comprising powdered charcoal and at least a kind of tea in a form of powder or extract, in addition to conventional components therefor.

2. Soap as claimed in Claim 1, which contains no artificial perfumery component.

3. Soap as claimed in claims 1 or 2, wherein said charcoal is selected from the group consisting of wood charcoal, coal, carbonized sawdust, carbonized coconut husk, and animal charcoal.

4. Soap as claimed in claims 1 to 3, wherein said charcoal is a carbomedicinalis .

5. Soap as claimed in claims 1 to 4, wherein said charcoal is composed in an amount of 0.1 -10 % by weight.

6. Soap as claimed in claims 1 to 5, wherein said tea is selected from the group consisting of green tea, black tea, oolong tea, *houttuynia cordata* tea, adley tea, mixture of coarse green tea and roasted whole rice, and pale tea.

7. Soap as claimed in claims 1 to 6, wherein said tea is in the form of dry powder, liquid extract or lyophilized powder of the extract.

8. Soap as claimed in claims 1 to 7, wherein said tea is composed in an amount of 0.1 - 5% by weight.

9. Use of a soap as claimed in any of the preceding claims for the preparation of a medicament for hair growth.

10. Use of a soap as claimed in any of claims 1 to 8 for providing more volume to the hair.

11. Use according to claim 10 wherein the soap is in form of a shampoo.
